**Europäisches Patentamt**

**European Patent Office**

**Office européen des brevets**

(19)

(11) Veröffentlichungsnummer: **0 040 836**
**A1**

# EUROPÄISCHE PATENTANMELDUNG

(12)

(21) Anmeldenummer: 81103953.6

(22) Anmeldetag: 22.05.81

(51) Int. Cl.³: **A 61 B 5/10**

(30) Priorität: 22.05.80 US 152514

(43) Veröffentlichungstag der Anmeldung: 02.12.81
Patentblatt 81/48

(84) Benannte Vertragsstaaten: AT BE CH DE FR GB IT LI NL SE

(71) Anmelder: SIEMENS AKTIENGESELLSCHAFT Berlin und München, Postfach 22 02 61, D-8000 München 22 (DE)

(72) Erfinder: Rüll, Hartwig, Dr., Balduin-Helm-Strasse 49, D-8080 Fürstenfeldbruck (DE)

(54) Fingerabdruckwandler und Verfahren zum Vergrössern von Fingerabdrücke.

(57) Es wird ein Fingerabdruckwandler beschrieben, welcher ein Abtastelement (2) mit einer Abtastfläche (3) zur Aufnahme eines Fingerabdrucks (8), eine Einrichtung (10, 11, 12, 13) zum Erzeugen einer elektrostatischen Aufladung auf der Abtastfläche (2) und eine Einrichtung (6, 16, 15; 20) zum Erwärmen der Abtastfläche (3) auf eine Temperatur umfaßt, die annähernd der Erweichungstemperatur des Abtastelements (2) entspricht. Es wird auch ein Verfahren zum Verstärken von Fingerabdrücken mit folgenden Verfahrensschritten beschrieben. Auf der Abtastfläche (3) wird ein Fingerabdruck (8) erzeugt. Die Abtastfläche (3) wird elektrostatisch aufgeladen. Die Abtastfläche (3) wird auf die Erweichungstemperatur erwärmt. Danach wird das Abtastelement (2) abgekühlt.

SIEMENS AKTIENGESELLSCHAFT     Unser Zeichen
Berlin und München            80 P 8216 E

0040836

## Fingerabdruckwandler und Verfahren zum Vergrößern von Fingerabdrücke

Die vorliegende Erfindung bezieht sich auf einen Fingerabdruckwandler nach dem Oberbegriff des Patentanspruchs 1 und auf ein Verfahren zum Vergrößern eines Fingeradrucks nach dem Oberbegriff des Patentanspruchs 15.

In einem Fingerabdruck-Eingabewandler oder -sensor wird der zu untersuchende Finger üblicherweise gegen eine Fläche gedrückt, beispielsweise eine Seite einer Glasplatte, und das Rippen- oder Furchenmuster des Fingers wird mittels einer Abfühleinrichtung, beispielsweise einen abfragenden Lichtstrahl, abgefühlt. Die Verarbeitung der auf diese Weise erhaltenen Fingerabdruckinformation kann mit Lasertechniken durchgeführt werden. Derartige Vorrichtungen zur Identifizierung eines Fingerabdrucks werden im allgemeinen dazu benutzt, den Zugriff von einzelnen Personen zu Informationen, beispielsweise zu Rechnerendgeräten (Informationszugriffskontrolle) oder zu Gebäuden (physische Zugriffskontrolle) benutzt.

Eines der Probleme im Zusammenhang mit Fingerabdrucksensoren betrifft die zuverlässige Abtastung von schwachen Fingerabdrücken. "Schwache Fingerabdrücke" sind Fingerabdrücke, die eine kleine Modulationstiefe auf der Sensorfläche haben, d.h. einen geringen topografischen Reliefwert. Ein schwacher Fingerabdruck kann auftreten, wenn der zu prüfende Finger nicht stark gegen die Fläche gedrückt wird, wenn der Finger nicht genügend Flüssigkeit, beispielsweise Öl oder Feuchtigkeit, aufweist, und dadurch keine ausreichenden Spuren auf der Fläche hinterläßt, usw..

Ed 1 Sti/28.1.1981

Es wird deshalb ein Fingerabdruckeingabesensor oder -wandler benötigt, welcher so ausgebildet ist, daß er nicht nur Fingerabdrücke gewöhnlicher Qualität, sondern auch schwache Fingerabdrücke geringer Modulationstiefe abtasten kann. Es besteht auch ein Bedarf für ein Verfahren zum Verstärken oder Vergrößern von Fingerabdrücken.

Aus der US-PS 4 053 228 geht ein Fingeridentifizierungssystem hervor, in welchem ein Fingerabdruck dadurch erzeugt wird, daß ein Finger gegen die Rückfläche einer transparenten Glasplatte gepreßt wird und der Finger in einer vorbestimmten Lage darauf gehalten wird. Dieser Fingerabdruck wird von einem durch die Vorderfläche der Glasplatte gestrahlten Lichtstrahl abgetastet oder abgefragt. Der abfragende Strahl wird teilweise an der Rückfläche der Glasplatte reflektiert und dadurch ein Signalstrahl erzeugt, welcher Fingerabdruckinformation trägt. Die Rückfläche der Glasplatte ist mit einem Überzug bedeckt, welcher dazu dient, den Unterschied im Reflexionsvermögen zwischen den Furchen und Rippen des Fingerabdrucks zu verstärken. Insbesondere verstärkt der Überzug auf der Rückfläche den Unterschied im Reflexionsvermögen auf dieser Fläche zwischen jenen Feldern, in denen die Rippen des Fingers in innigem Kontakt mit der Rückfläche der Platte stehen und jenen Feldern unter den Furchen des Fingers, in denen Luft mit der Rückfläche der Platte in Kontakt steht. In diesem Fingerabdruckwandler wird ein verstärkter Fingerabdruck nur so lange erzeugt, wie der Finger die Rückfläche der Platte berührt,und zwar kurzzeitig.

Aus der US-PS 4 120 585 ist ein nachgiebiges,elastisches Prisma für den Gebrauch in einem optischen Abbildungssystem, z.B. einem Fingerabdruckleser, bekannt geworden. Die Basisfläche des Prismas wird von dem zu untersuchenden Finger berührt. Das nachgiebige Prisma verformt sich unter dem Druck des berührenden Fingers. Licht aus einer Lichtquelle durchstrahlt eine Seitenfläche des Prismas in Richtung Basisfläche, wo es reflektiert und durch die andere Seitenfläche in Richtung eines lichtempfindlichen Elements strahlt. Dieses Element ist zum Auslösen oder Betätigen des optischen Abbildungssystems vorgesehen. Wenn ausreichender Fingerdruck vorhanden ist, wird zwischen der Basisfläche und einem Gehäuse ein Luftspalt erzeugt, der in einem zusätzlichen Feld resultiert, dessen Lichtstrahl zum lichtempfindlichen Element gerichtet wird. Dieses Element aktiviert das System, indem es einen Schalter schließt. Bei diesem Fingeradrucksystem werden keine Einrichtungen zum Verarbeiten schwacher Fingerabdrücke erörtert.

Aus SPIE Vol. 185, Optical Processing Systems (1979) Seiten 86 bis 92 ist eine elastomere Speichervorrichtung bekannt geworden, die einen Fotoleiter benutzt. Ein Glassubstrat wird zur Bildung einer transparenten leitenden Grundebene chemisch geätzt. Die Grundfläche wird mit dem erwähnten Fotoleiter bedeckt und mit einer dünnen elastomeren Schicht überzogen. Mehrere zehn Mikrometer (mehrere mils) über der elastomeren Oberfläche und parallel dazu ist eine Ladungsfläche, insbesondere -ebene, angeordnet. Der daraus resultierende Spalt ist mit Argon unter niedrigem Druck gefüllt. Während des Betriebes ist zwischen der Ladungsfläche und der Grundfläche eine Hochspannungsquelle geschaltet, die eine quer zu der elastomerischen oder fotoleitenden Schicht gerichtete elektrostatische Kraft erzeugt. Die elektrostatische Kraft steht senkrecht auf der elastomeren Oberfläche und ist in jedem Punkt der elektrischen Feldstärke proportional. Weil das Elastomer inkompressibel und die Kraft gleichmäßig ist, tritt keine Verformung auf. Wenn der Fotoleiter einer Lichtverteilung ausgesetzt wird, werden Ladungsträger foto-erzeugt und bewegen sich in dem elektrischen Feld in Richtung zur Grenzfläche zwischen Elastomer und Fotoleiter, wo sie aufgefangen werden. Die aufgefangenen Ladungen bilden eine elektrostatische Oberflächenladungsschicht, in welcher die Ladungsdichteverteilung direkt proportional zur Belichtungsverteilung des Eingangssignals ist. Die resultierende elektrische Feldverteilung bewirkt eine Verformung des Elastomers, wobei ein Oberflächenreliefmuster erzeugt wird, welches in direktem Bezug zur Belichtungsverteilung durch die Oberflächenladungsdichte steht. Die Verformung setzt sich so lange fort, bis die ungleichmäßige Ladungsverteilung beibehalten wird. Diese Speichervorrichtung ist übrigens nicht zur Verwendung als ein Fingerabdruckwandler in Betracht gezogen worden.

Aus SPIE, Vol. 123, Optical Storage Materials and Methods (1977) Seiten 32 bis 36 ist ein thermoplastisches Datenspeichermedium bekannt geworden. Dieses Medium ist eine mehrschichte Vorrichtung, die aus einer thermoplastischen, einer fotoleitenden und einer transparenten leitenden Schicht auf einem Glassubstrat oder flexiblen Polyestersubstrat besteht. Die optische Datenspeicherung in Thermoplasten basiert auf dem Prinzip, daß sich ein Thermoplast unter Druck verformt, wenn es auf eine geeignete Temperatur erhitzt wird. Während des Einschreibens wird eine gleichmäßige Ladung auf der thermoplastischen Oberfläche erzeugt. Dann wird die Vorrichtung mit einem holografischen Muster belichtet, welches die Leitfähigkeit des Fotoleiters und folglich die Oberflächenladungsverteilung verändert. Diese ungleichförmige Ladungsverteilung hat elektrostatische Kräfte zur Folge, welche den Thermoplasten bei Erwärmung auf eine kritische Erweichungs- oder Entwicklungstemperatur verformen. Wenn die Probe abkühlt, bleibt die dem holografischen Muster entsprechende Verformung erhalten und die Information kann durch Beleuchtung mit einem Referenzstrahl wiedergegeben werden. Auch dieses Speichermedium ist für eine Anwendung als ein Fingerabdruckwandler nicht vorgesehen worden.

Ähnliche optische Aufzeichnungs- und Speichervorrichtungen sind aus SPIE, Vol. 123, Optical Storage and Methods (1977) S. 10 bis 16 und S. 74 bis 77 bekannt geworden.

Der Erfindung liegt die Aufgabe zugrunde, einen Fingerabdruckeingabesensor oder -wandler der eingangs genannten Art zu schaffen, welcher auch schwache Fingerabdrücke, die nur eine kleine Modulationstiefe aufweisen, festzustellen und abzufühlen vermag bzw. ein entsprechendes Verfahren anzugeben.

Diese Aufgabe wird durch die im kennzeichnenden Teil des Patentanspruchs 1 bzw. 16 angegebenen Merkmale gelöst.

Bevorzugte und vorteilhafte Ausführungsformen der Erfindung gehen aus den Unteransprüchen 2 bis 15 bzw. 17 bis 19 hervor.

Vorteile der Erfindung sind darin zu sehen, daß der erfindungsgemäße Fingerabdruckwandler bzw. das erfindungsgemäße Verfahren  das auf einer Oberfläche erzeugte Relief eines Fingerabdrucks verstärkt und daß der Fingerabdruck eine kurze Zeit lang gespeichert werden kann.

Der erfindungsgemäße Fingerabdruck-Eingabewandler umfaßt gemäß den Ansprüchen ein Abtastelement, das eine Abtastfläche zur Aufnahme eines Fingerabdrucks, eine Einrichtung zum Erzeugen einer elektrostatischen Aufladung der Abtastfläche und eine Heizeinrichtung zum Erwärmen der Abtastfläche des Abtastelements auf eine Temperatur, die etwa der Erweichungstemperatur des Materials des Abtastelements entspricht, aufweist.

Die Erweichungstemperatur muß höher sein als die Umgebungstemperatur des Fingerabdruck-Eingabewandlers.

Generell sollte die Erweichungstemperatur des Abtastelements über 40°C liegen.

Das Abtastelement kann aus einem elastischen oder nachgiebigen Material bestehen. Vorzugsweise ist es aus
einem elastomerischen oder thermoplastischen Material
gefertigt, beispielsweise aus plastifiziertem Polystyrol.
Es kann auch aus einem Material gefertigt sein, dessen
Eigenschaften jenen eines elastomerischen Materials
ähnlich sind. Unter einem elastomerischen Material
sei jedes Polymer verstanden, welches die elastischen
Eigenschaften von natürlichem Gummi aufweist. Speziell
kann ein elastomerisches Material von vergleichsweise
niedriger Erweichungstemperatur gewählt werden.

Bei einer bevorzugten Ausführungsform des Fingerabdruck-
Eingabewandlers ist die Erweichungstemperatur so gewählt,
daß sie nur wenige Grade Celsius über der Temperatur des
menschlichen Körpers liegt.

Beim Betrieb des Eingabewandlers wird in einem ersten
Schritt der zu überprüfende Finger gegen die Oberfläche
des Abtastelements gedrückt, so daß er ein Oberflächenrelief hinterläßt. Abhängig von dem Druck des Fingers
und dem Grad des hinterlassenen Öls bzw. der hinterlassenen Feuchtigkeit wird die Modulationstiefe des
Fingerabdrucks für gewöhnliche Abtastung der Oberfläche mehr oder weniger ausreichend. Die Oberfläche
des Abtastelements wird dann mittels der Aufladungseinrichtung elektrostatisch aufgeladen. Aufgrund des
Reliefs der Oberfläche wird die Ladungsverteilung
nicht homogen. Die Ladungsverteilung repräsentiert
genau das Bild der Reliefstruktur. Danach wird das
Abtastelement, zumindest aber seine Abtastfläche oder
seine Oberfläche, auf eine Temperatur erwärmt, welche
in der Nähe der Erweichungstemperatur liegt, wodurch
das Abtastelement auf seiner Abtastfläche weich wird.

0040836

Das Material des erweichten Abtastelements folgt der durch das elektrische Feld ausgeübten elektrischen Kraft, welche entsprechend der lokal variierenden Ladungsdichte von Flächenelement zu Flächenelement verschieden ist. Mit anderen Worten, die Oberfläche wird in gewissen Flächenelementen angehoben und in anderen Flächenelementen abgesenkt. Dieses Anheben und Absenken der Oberfläche hat die Tendenz, das Relief der Oberfläche zu verstärken und den Modulationsgrad zu erhöhen. Anders ausgedrückt: Die Täler des Fingerabdruckmusters auf der Oberfläche werden tiefer und die Rippen oder Rücken werden höher. Auf diese Weise er- oder enthält die geriffelte Oberfläche des Abtastelements ein deutlicheres topografisches Relief . Danach wird die Abtast- oder Sensorfläche abgekühlt, so daß das verstärkte Relief "eingefroren" wird.

Die Ladung kann vorzugsweise durch eine Koronabeladung auf die Oberfläche aufgebracht werden. Zu diesem Zweck kann die Aufladungsvorrichtung einen Draht umfassen, welcher in einem Abstand von der Oberfläche des Abtastelements angeordnet ist, sowie eine Spannungsquelle zum Anlegen einer Gleichspannung zwischen dem Draht und der Oberfläche des Abtastelements. Die Aufladungseinrichtung kann auch eine nahe bei der Oberfläche des Abtastelements angeordnete, flächige Elektrode in Form eines Drahtgitters und eine Spannungsquelle zum Anlegen einer Gleichspannung zwischen der Elektrode und dem Abtastelement umfassen.

Die Heizeinrichtung kann eine elektrische Heizeinrichtung sein. Sie kann einen Heizwiderstand enthalten, der beispielsweise aus einem Widerstandsdraht aus einem flächigen Drahtgitter oder aus einer transparenten Elektrode besteht, die einen geeigneten Flächenwiderstand aufweist. Während des Betriebs fließt ein Heizstrom durch den Heizwiderstand. Das Drahtgitter kann im Hinblick auf die Wellenlänge des abtastenden Lichtstrahls transparent sein.
Um eine gute Wärmekopplung zu erreichen, wird der Heizwiderstand zweckmäßigerweise an dem Abtastelement angebracht.

Die Heizeinrichtung braucht aber keine ohm'sche Heizeinrichtung oder Widerstandsheizeinrichtung zu sein.
Das Erwärmen kann auch durch Strahlung erreicht werden.
Beispielsweise kann die Wärme mittels eines Infrarot-
Licht (IR)-Lasers, speziell einer IR LED, mittels
eines Halbleiterlasers oder mittels ähnlicher IR-Lichtquellen auf die Abtastfläche übertragen werden.

Die in dem Sensorelement gespeicherte Fingerabdruckinformation kann entweder mit optischen Mitteln, beispielsweise durch Totalreflexion oder durch Laseroptik
in für sich bekannter Weise ausgelesen werden. Das
topografische Relief kann auf irgend eine bekannte Weise
analysiert werden.

Ein besonderer Vorteil der Erfindung liegt darin, daß
das verstärkte Relief auf der Oberfläche des Abtastelements stabil oder "eingefroren" bleibt, nachdem
die Heizeinrichtung abgeschaltet worden ist und das
Abtastelement sich abgekühlt hat.

Um das Relief zu löschen, wird die Abtastfläche wieder
aufgeheizt, ohne daß die Oberfläche elektrostatisch aufgeladen wird. Beispielsweise kann wieder der Heizstrom
durch den Heizwiderstand geschickt werden, welcher
thermisch mit dem elastomerischen Material verbunden
ist. Beim Wiederaufheizen der Abtastfläche wird die
Oberfläche wieder weich und die schmelzende Oberfläche
wird aufgrund der Oberflächenspannung wieder geglättet.
Der Wandler wird auf diese Weise für einen neuen Fingerabdruck-Identifizierungszyklus präpariert. Beim Löschen
des Reliefs sollte die Heiztemperatur höher liegen als
beim vorangegangenen Verstärken des Oberflächenreliefs.

Ein erfindungsgemäßes Verfahren zum Verstärken eines
Fingerabdrucks umfaßt folgende Verfahrensschritte:
Es wird auf der Abtastfläche eines Abtastelements
ein Fingerabdruck erzeugt, die Abtastfläche wird
elektrostatisch aufgeladen, die Abtastfläche wird
auf eine Temperatur erwärmt, die ungefähr der Erweichungstemperatur des Materials entspricht, und das Abtastelement wird auf eine Temperatur abgekühlt, welche unterhalb der Erweichungstemperatur liegt.

Ausführungsbeispiele der Erfindung sind in den Figuren
dargestellt und werden in der nun folgenden Beschreibung,
aus welcher weitere Vorteile und Eigenschaften der Erfindung hervorgehen, näher erläutert. Von den Figuren
zeigen:

Figuren 1 bis 8 eine Ausführungsform eines erfindungsge-
         mäßen Fingerabdruckwandlers in verschie-
         denen Betriebszuständen,

Figur 9 einen erfindungsgemäßen Fingerabdruckwandler,
         der durch eine IR-Lichtquelle aufheizbar ist,

Figur 10 eine Draufsicht auf eine Elektrode, die in
         einem erfindungsgemäßen Fingerabdruckwandler
         benutzt werden kann,

Figur 11 eine perspektivische Darstellung eines erfindungsgemäßen Fingerabdruckwandlers mit einer
         gitterartigen Beladungselektrode, und

Figur 12 eine perspektivische Darstellung eines erfindungsgemäßen Fingerabdruckwandlers mit einem
         Einzeldraht als Beladungselektrode.

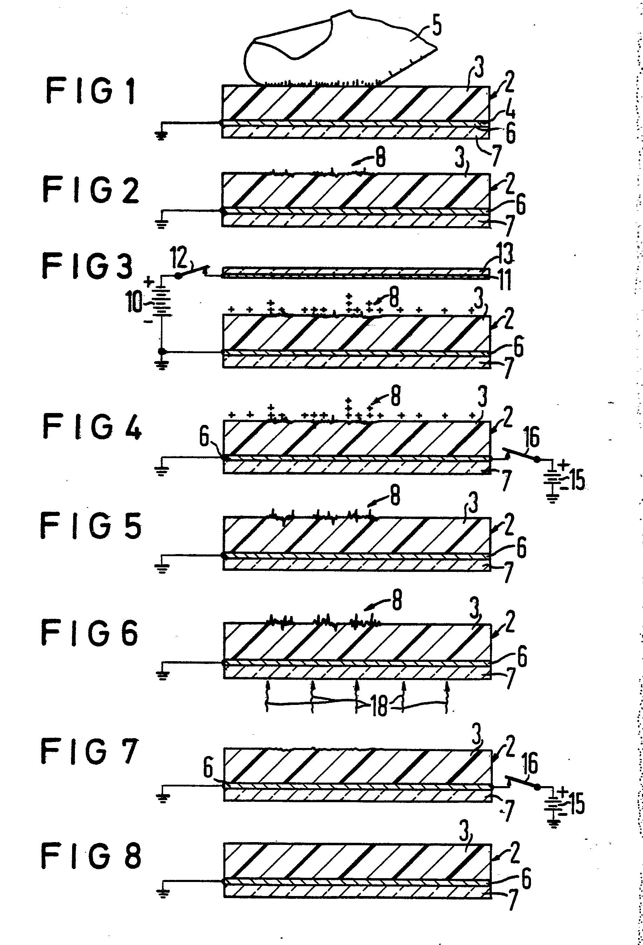

Gemäß der Figur 1 ist eine rechtwinkelige flache Platte als ein Abtastelement 2 vorgesehen. Das Abtastelement 2 weist eine erste Oberfläche 3, die eine Abtastfläche bildet, und eine zweite Oberfläche 4 auf. Das Abtastelement 2 ist vorzugsweise aus einem wieder verwendbaren elastischen Material, vorzugsweise aus einem elastomeren oder thermoplastischen Material, wie beispielsweise plastifiziertes Polystyrol gefertigt. Das Material des Abtastelements 2 ist elektrisch nicht leitend. Es ist so gewählt, daß seine Erweichungstemperatur höher ist als die das Element 2 umgebende Umgebungstemperatur. Insbesondere sollte die Erweichungstemperatur höher gewählt werden als 40°C. Sie kann so gewählt werden, daß sie nur einige wenige Grad Celsius über der Umgebungstemperatur liegt. Die Abtastfläche 3 ist so ausgebildet, daß sie für eine kurze Zeitdauer den Druck eines zu überprüfenden Fingers empfängt. Abweichend von der Darstellung in Figur 1 muß die Abtastfläche 3 des Abtastelements 2 nicht flach sein. Sie kann auch in der Weise gekrümmt sein, daß sie dem Umriß des Fingers 5 angepaßt ist.

An der zweiten Oberfläche 4 ist eine Elektrode 6 angebracht. Die Elektrode 6 ist vorzugsweise aus einem Material gefertigt, welches hinsichtlich des Lichts eines Abfragestrahls (siehe Figur 6) transparent ist. Sie kann aus Indiumoxid (InO) gefertigt sein. Die Elektrode 6 weist die Form eines dünnen rechtwinkeligen Platte oder eines Drahtsystems auf oder kann auch ein dünner Filmüberzug sein. Die Elektrode 6 kann mit Masse verbunden sein. Sie wird von einer lichttransparenten Trägerplatte 7, beispielsweise ein Glassubstrat, gestützt.

In einem ersten Arbeitsschritt wird der Finger 5 gegen die flache Abtastfläche 3 des Abtastelements 2 gedrückt. Dies kann der Figur 1 entnommen werden.

Der zweite Arbeitsschritt ist durch die Fortnahme des Fingers 5 von der Abtastfläche 3 gegeben und in der Figur 2 dargestellt. Auf der Fläche 3 ist ein Fingerabdruck 8 zurückgeblieben. Aufgrund eines kleinen Druckes oder einer abgenutzten Fingerabdruckstruktur kann der Fingerabdruck nur schwach sein. Im Laufe der folgenden Arbeitsschritte wird sogar ein schwacher Fingerabdruck 8 verstärkt, so daß dieVerarbeitung der Fingerabdruckinformation möglich ist.

Es sei darauf hingewiesen, daß die Abtastfläche 3 durch den Finger 5 eingedrückt werden kann. Wenn der Finger 5 auf der Fläche 3 ruht, erhält sie eine nicht ebene Flächenstruktur. Aufgrund des Druckes und der Wärme des Fingers 5 paßt sich die Oberfläche 3 der Flächenstruktur des Fingers 5 an. Dies ähnelt dem Verfahren des Prägens oder Münzprägens. Für die Zwecke der vorliegenden Ausführungsform reicht es aus, wenn das Abtastelement 2 einen "kurzzeitigen Fingerabdruckspeicher" bildet, der seinen ursprünglichen Zustand, d.h. eine ebene Fläche 3 nach einer gewissen Zeit zurückerhält.

Der dritte Arbeitsschritt des Fingerabdrucksensors umfaßt eine elektrostatische Aufladung und ist in der Figur 3 dargestellt. Zu diesem Zweck ist eine Einrichtung zum elektrostatischen Aufladen vorgesehen. Diese Einrichtung enthält eine Spannungsquelle 10, die eine höhere Spannung V erzeugt, welche elektrisch zwischen Masse und einer Elektrode 11 mittels eines Schalters 12 anlegbar ist. Die Elektrode 11 ist in einem gewissen Abstand von der Abtastfläche 3 angeordnet. Sie wird von einem Trägerelement 13 getragen, beispielsweise von einem Glassubstrat. Die Elektrode 11 kann ein einfacher Draht sein, der parallel zur Fläche 3 angeordnet ist. In der vorliegenden Ausführungsform besteht sie aus einer planen Elektrode, welche mit der Unterseite oder unteren Fläche des Trägerelements 13 verbunden ist.

Sobald der Schalter 12 geschlossen ist, bewirkt die hohe Spannung V zwischen den Elektroden 6 und 11 eine Ionisation der Luft in der Nähe der Elektrode 11. Aufgrund der Ionisation der Luft werden elektrische Ladungen zur Abtastfläche 3 geleitet. Diese Ladungen werden auf der Fläche 3 zurückgehalten. Sie können von der Fläche 3 nicht fortfließen, weil das Abtastelement 2 aus einem elektrischen Isolator besteht.

Wie ebenfalls in der Figur 3 dargestellt, ist der positive Anschluß der Hochspannungsquelle 10 mit der Elektrode 11 verbunden, wohingegen der negative Anschluß mit der Elektrode 4 verbunden ist. Jedoch ist auch eine Aufladung mit negativen Teilchen möglich. Um eine negative elektrostatische Aufladung zu erhalten, braucht nur die Polarität der Hochspannungsquelle 10 im Hinblick auf die Elektroden 4 und 11 vertauscht zu werden. Wie in der Figur 3 angedeutet, sammeln sich die positiven Teilchen vorzugsweise auf gewissen Oberflächenelementen im Bereich des Fingerabdrucks 8. Aufgrund der lokal variierenden Ladungsdichte sind unterschiedliche elektrische Feldstärken vorhanden und es werden unterschiedliche elektrische Kräfte an unterschiedlichen Oberflächenelementen ausgeübt.

In der Figur 4 ist der vierte Schritt bei der Betätigung des Fingerabdruckwandlers dargestellt. Dieser vierte Schritt besteht im Aufheizen der Oberfläche 3. Er kann mit einem "Entwicklungsvorgang" hinsichtlich des Fingerabdrucks verglichen werden. Das Aufwärmen wird mittels einer Heizeinrichtung durchgeführt, welche in der vorliegenden Ausführungsform elektrischer Natur ist, insbesondere in Form einer Widerstandsheizeinrichtung. Die Heizeinrichtung enthält eine Heizstromquelle

0040836

15 und einen Schalter 16. Die Heizstromquelle 15 und der Schalter 16 sind zwischen einem Ende der Elektrode 6 und Masse in Reihe geschaltet. Das andere Ende der Elektrode 6 ist ebenfalls mit Masse verbunden. Es sei darauf hingewiesen, daß die Aufladungseinrichtung 10 bis 13 abgeschaltet ist. Beim Schließen des Schalters 16 gibt die Heizstromquelle 15 einen Heizstrom (Gleich- oder Wechselstrom oder gepulsten Strom) ab, der durch die Elektrode 6 fließt. Demgemäß ist die Elektrode 6 nicht nur eine Hochspannungselektrode, die zur elektrostatischen Aufladung benutzt wird. Sie ist gleichzeitig ein Heizwiderstand, beispielsweise ein Widerstandsgitter. Der Heizstrom erwärmt das Abtastelement 2. Der Heizstrom ist betragsmäßig so gewählt, daß er eine Temperatur an der Abtastfläche 3 erzeugt, die wenigstens annähernd der Erweichungstemperatur des elastomerischen oder thermoplastischen Materials entspricht. Im Laufe des Erwärmungsprozesses wird die Fläche 3 des Abtastelements 2 weich und beginnt fast zu schmelzen. Aufgrund der über dem Fingerabdruck 8 lokal variierenden elektrischen Kräfte werden die Rippen in Richtung Elektrode 11 angehoben. Folglich wird das topografische Relief des Fingerabdrucks 8 auf der Fläche 3 verstärkt. Die Verstärkung entspricht der lokalen elektrischen Feldverteilung auf der Fläche 3.

In der Figur 5 ist das verstärkte Muster des Fingerabdrucks 8 dargestellt.

Nach der Figur 5 besteht der fünfte Arbeitsschritt im Abkühlen des Abtastelements 2. Die Abkühlung kann dadurch erreicht werden, daß einfach der Schalter 16 geöffnet wird, vorausgesetzt, daß die Umgebungstemperatur niedrig genug ist. Zusätzlich kann eine Kühlvorrichtung (nicht dargestellt) vorgesehen sein, beispielsweise kann ein thermoelektrisches Peltier-Element mit dem Abtastelement 2 verbunden sein. Das Abkühlen des Abtastelements 2 hat ein "Einfrieren" des verstärkten Reliefs des Flächenbereichs 8 zur Folge. Es sei darauf hingewiesen, daß während des Abkühlvorgangs die Elektrode 11 von der Hochspannungsquelle 10 abgeklemmt ist.

In der Figur 6 ist der sechste Arbeitsschritt dargestellt. In diesem Schritt wird die in dem "gefrorenen" Fingerabdruck 8 enthaltene Information ausgelesen und analysiert. Dies kann mit einem abfragenden oder lesenden Strahl 18 durchgeführt werden. Dieser Strahl 18 kann auf die untere Fläche 4 des Abtastelements 2 gerichtet werden. Das Licht 18 wird durch das transparente Element 7 und die transparente Elektrode 6 in das Abtastelement 2 übertragen. Letztlich erreicht es die obere Fläche 3. Nachdem es den Fingerabdruck 8 passiert hat, trägt es die Fingerabdruckinformation. Das Licht ist entsprechend den Furchen und Rippen der Fläche 3 räumlich moduliert, d.h. es trägt die Information über die geometrische Struktur des Fingersabdrucks 8. Die Analysierung der Information, die in dem modulierten Licht enthalten ist, kann in bekannter Weise durchgeführt werden.

Die Figur 7 zeigt den siebten Arbeitsschritt. In diesem Schritt wird das Abtastelement 2 erneut erwärmt. Die Erwärmung wird auch in diesem Fall durch Schließen des Schalters 16 erreicht. Da die Elektrode 11 keine positiven Teilchen in Richtung Fläche 3 mehr sendet, können sich auf den Rippen des Fingerabdrucks 8 keine Teilchen anhäufen. Sobald die Erweichungstemperatur des Abtastelements 2 erreicht oder überschritten wird, wird die Fläche 3 wieder flach oder eben. Mit anderen Worten: die im Fingerabdruck 8 enthaltene Information wird ausgelöscht, d.h. das Oberflächenrelief wird ausgelöscht.

Figur 8 zeigt den achten und letzten Arbeitsschritt.

In diesem Schritt wird das Abtastelement 2 durch Öffnen des Schalters 16 wieder abgekühlt. Zu dieser Zeit wird die ebene Fläche 3 "eingefroren". Das Abtastelement 2 ist jetzt für den nächsten Fingerabdruckvorgang und Lesezyklus bereit.

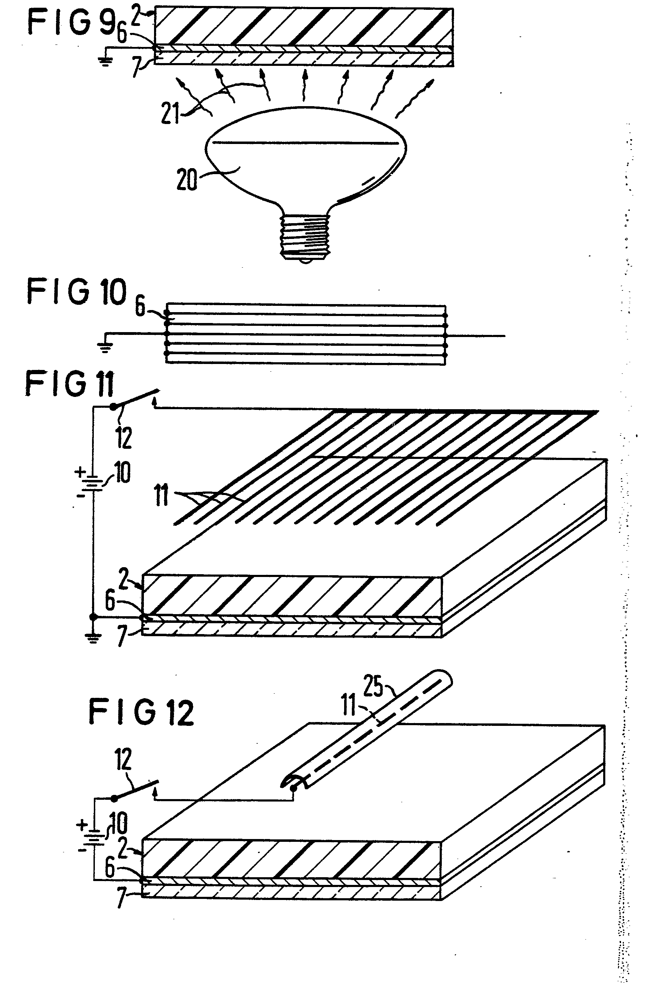

Aus der Figur 9 geht hervor, daß das Erwärmen des Abtastelements 2 (siehe Figuren 4 und 7) auch durch eine strahlende Heizquelle erfolgen kann. In diesem Fall wird eine IR-Strahlungsquelle 20 benutzt, welche IR-Strahlung 21 in Richtung der unteren Seite der Elektrode 6 aussendet. Die IR-Strahlungsquelle 20 kann eine einzelne IR-Quelle oder ein Feld aus einzelnen IR-Quellen sein. Als IR-Quelle 20 kann auch eine IR-Strahlung imittierende LED oder IR-Lampe benutzt werden.

In der Figur 10 ist eine Ausführungsform der Elektrode 6 dargestellt. Die Elektrode kann aus einer Anordnung paralleler Widerstandsdrähte bestehen. Diese Drähte können sowohl als Elektrode während des elektrostatischen Aufladungsvorgangs als auch als Heizung während des Erwärmungsvorgangs dienen.

In der Figur 11 ist eine perspektivische Ansicht einer anderen Ausführungsform der Erfindung dargestellt. In dieser Ausführungsform besteht die Elektrode 11 zum Beladen der oberen Fläche des Elements aus einer Anordnung paralleler Drähte , welcher auf einer Seite mit einem Verbindungselement 23 verbunden sind, das seinerseits über den Schalter 12 mit der Hochspannungsquelle 10 verbunden ist. Die Anordnung der parallelen Drähte kann von einer geeigneten Vorrichtung (nicht dargestellt) gehalten werden.

In der Figur 12 ist eine perspektivische Ansicht einer weiteren Ausführungsform der Erfindung dargestellt. In dieser weiteren Ausführungsform besteht
die Elektrode 11 aus einem einzigen Draht, der parallel
zur Oberfläche des Elements 2 gehalten ist. Die Elektrode 11 ist von der Fläche durch einen Schirm 25 abgeschirmt. Deshalb werden geladene Teilchen im wesentlichen
zwischen dem Schirm 25 und der oberen Fläche des Abtastelements 2 erzeugt.


19 Patentansprüche
12 Figuren

Patentansprüche

1. Fingerabdruckwandler g e k e n n z e i c h n e t
d u r c h

a) ein, eine zum Aufdrücken eines Fingerabdrucks (8)
vorgesehene Abtastfläche (3) aufweisendes Abtastelement (2), das aus einem, eine höher als die Umgebungstemperatur liegende Erweichungstemperatur aufweisenden Material gefertigt ist,

b) eine Einrichtung (10, 11, 12, 13) zum Erzeugen einer
-elektrostatischen Aufladung der Abtastfläche (3) und
durch

c) eine Einrichtung (6, 15, 16; 20) zum Aufwärmen
der Abtastfläche (3) auf etwa die Erweichungstemperatur des Materials des Abtastelements (2).

2. Wandler nach Anspruch 1, d a d u r c h   g e -
k e n n z e i c h n e t , daß das Material des Abtastelements (2) aus einem elastischen oder nachgebigen
Material besteht.

3. Wandler nach Anspruch 2, d a d u r c h   g e -
k e n n z e i c h n e t , daß das Material des Abtastelements (2) aus einem elastomerischen, elastomerähnlichem oder thermoplastischen Material besteht.

4. Wandler nach Anspruch 2 oder 3, d a d u r c h
g e k e n n z e i c h n e t , daß das Material des
Abtastelements (2) aus einem plastifizierten Polystyrol
oder aus natürlichem Gummi besteht.

5. Wandler nach einem der vorhergehenden Ansprüche,
d a d u r c h   g e k e n n z e i c h n e t , daß
die Erweichungstemperatur des Materials des Abtastelements (2) nur wenige Celsiusgrade über der Temperatur
des menschlichen Körpers liegt.

6. Wandler nach einem der vorhergehenden Ansprüche, d a d u r c h   g e k e n n z e i c h n e t , daß das Abtastelement (2) ein Flachmaterial bildet.

7. Wandler nach einem der vorhergehenden Ansprüche, d a d u r c h   g e k e n n z e i c h n e t , daß die Einrichtung (10, 11, 12, 13) zum Erzeugen einer elektrostatischen Aufladung eine mit Abstand vor der Abtastfläche (3) angeordnete erste Elektrode (11) und eine Spannungsquelle (10) zum Erzeugen einer Hochspannung zwischen der ersten Elektrode (11) und der Abtastfläche (3) aufweist.

8. Wandler nach Anspruch 7, d a d u r c h   g e - k e n n z e i c h n e t , daß das Abtastelement (2) einen Flächenbereich aufweist, auf den eine zweite Elektrode (6) aufgebracht ist und daß die Spannungsquelle (10) zwischen der ersten und zweiten Elektrode (11 bzw. 6) geschaltet ist.

9. Wandler nach Anspruch 8, d a d u r c h   g e - k e n n z e i c h n e t , daß das Abtastelement (2) aus einer flachen Platte mit einer ersten und zweiten Seite besteht, daß die erste Seite die Abtastfläche (2) bildet, daß die zweite Seite den Flächenbereich bildet, auf den die zweite Elektrode (6) aufgebracht ist, daß die erste Elektrode (11) aus einem Draht besteht, der parallel zur Abtastfläche (2) angeordnet ist und daß die zweite Elektrode (6) eine Flächenelektrode bildet, welche im wesentlichen die zweite Seite der Platte bedeckt.

10. Wandler nach Anspruch 9, d a d u r c h   g e - k e n n z e i c h n e t , daß die zweite Elektrode (6) zumindest teilweise für einen in Betracht kommenden Lichtstrahl transparent ausgebildet ist.

11. Wandler nach Anspruch 10, d a d u r c h  g e -
k e n n z e i c h n e t , daß die zweite Elektrode (6)
aus einem Drahtgitter besteht.

12. Wandler nach Anspruch 7 oder nach Anspruch 7 und
einem der Ansprüche 8 bis 11, d a d u r c h  g e -
k e n n z e i c h n e t , daß die erste Elektrode (11)
aus einem flächigen Drahtgitter
besteht.

13. Wandler nach einem der vorhergehenden Ansprüche,
d a d u r c h  g e k e n n z e i c h n e t , daß
die Einrichtung (6, 15, 16) zum Erwärmen der Abtastfläche (3) einen Heizwiderstand aufweist, welcher
wärmeleitend mit dem Abtastelement (2) verbunden ist,
sowie eine Heizstromquelle (15, 16) zum Durchleiten
eines Heizstromes durch den Heizwiderstand.  -

14. Wandler nach Anspruch 8 und 13, d a d u r c h
g e k e n n z e i c h n e t , daß die zweite Elektrode
(6) zugleich als der Heizwiderstand ausgebildet ist.

15. Wandler nach einem der vorhergehenden Ansprüche,
d a d u r c h  g e k e n n z e i c h n e t , daß
die Einrichtung (20) zum Erwärmen der Abtastfläche (3)
eine Infrarotstrahlungsquelle aufweist, die in einem
Abstand von dem Abtastelement (2) angeordnet ist.

16. Verfahren zum Verstärken eines Fingerabdruckes,
g e k e n n z e i c h n e t  d u r c h  folgende
Verfahrensschritte:
   a') es wird ein Fingerabdruck auf einer Abtastfläche
       (2) eines Abtastelementes (3) erzeugt;
   b') die Abtastfläche (3) wird elektrostatisch aufgeladen;
   c') die Abtastfläche (3) wird auf eine Temperatur erwärmt, welche annähernd einer Erweichungstemperatur
       eines Materials des Abtastelements (2) entspricht,

0040836

wobei die elektrostatische Ladung auf der Abtastfläche (3) beibehalten wird; und

d') das Abtastelement (2) wird auf eine Temperatur abgekühlt, welche unterhalb der Erweichungstemperatur
liegt.

17, Verfahren nach Anspruch 16, d a d u r c h   g e -
k e n n z e i c h n e t , daß der Fingerabdruck
analysiert wird, bevor das Abtastelement (2) abgekühlt
worden ist.

18. Verfahren nach Anspruch 16 oder 17, d a d u r c h
g e k e n n z e i c h n e t , daß nach dem Abkühlen des
Abtastelements (2) dieses wieder auf eine Temperatur
erwärmt wird, welche etwa der Erweichungstemperatur
des Materials dieses Elements entspricht, wobei aber
die elektrostatische Aufladung angehalten wird, und
daß danach die Abtastfläche (3) auf eine unterhalb
der Erweichungstemperatur liegende Temperatur abgekühlt wird.

19. Verfahren nach einem der Ansprüche 16 bis 18,
d a d u r c h   g e k e n n z e i c h n e t , daß
die Abtastfläche (3) auf die Umgebungstemperatur
abgekühlt wird.

0040836

80P 8216 DE

FIG 1

FIG 2

FIG 3

FIG 4

FIG 5

FIG 6

FIG 7

FIG 8

FIG 9

FIG 10

FIG 11

FIG 12

# 0040836

**Europäisches Patentamt**

## EUROPÄISCHER RECHERCHENBERICHT

EP 81 10 3953

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 532 426 (C.G. LEMMOND)<br>* Zusammenfassung; Spalte 4, Zeile 9 - Spalte 5, Zeile 37; Spalte 6, Zeilen 6-16;Abbildungen * | 1-3,<br>5-7,<br>13,16<br>17-19 |
| | -- | |
| | GB - A - 900 068 (GENERAL ELEC-TRIC)<br>* Patentansprüche; Abbildung 1; Seite 2, Zeile 40 - Seite 3, Zeile 110 * | 1-3,<br>6-9,<br>16 |
| | -- | |
| A | US - A - 3 982 836 (H. GREEN)<br>* Zusammenfassung; Abbildungen * | 1,16 |
| | -- | |
| A | US - A - 3 549 253 (I. BRODIE)<br>* Zusammenfassung; Abbildungen * | 1 |
| | ---- | |

### KLASSIFIKATION DER ANMELDUNG (Int. Cl.³)

A 61 B 5/10

### RECHERCHIERTE SACHGEBIETE (Int. Cl.³)

A 61 B 5/10
G 07 C 11/00
G 06 K 9/00
9/20
G 03 G 5/022
B 41 M 5/26

### KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 01-09-1981 | DAVID |

EPA form 1503.1   06.78